# EUROPEAN PATENT APPLICATION

(11) **EP 2 145 647 A1**
(43) Date of publication of application: **20.01.2010**
(21) Application number: 09007586.2
(22) Date of filing: 09.06.2009
(51) Int. Cl.: A61M 39/04, A61M 39/22

(54) **Liquid coinfusion unit**

(30) Priority: 16.07.2008 JP 2008184582
(71) Applicant: Tyco Healthcare Group LP, Mansfield, MA 02048 (US)
(72) Inventor: Satoh, Takashi, Fukuroi-shi 437-0004 Shizuoka (JP); Funamura, Shigeaki, Fukuroi-shi 437-0004 Shizuoka (JP); Kitani, Ichiro, Fukuroi-shi 437-0004 Shizuoka (JP)
(74) Representative: Olsson, Carl H.S.

(57) **Abstract**

[Problem] To provide a liquid coinfusion unit inside which liquid and air are unlikely to accumulate.

[Resolving Means] A liquid coinfusion unit A consists of a chamber part 11, a main upstream branch pipe 12, a downstream branch pipe 13, a supplementary upstream branch pipe, and a stopcock 30 which can allow communication/block the area between the chamber part 11 and main upstream branch pipe 12, and the area between the chamber part 11 and downstream branch pipe 13, respectively. The portion of the liquid flow channel inside the chamber part 11 which runs from the main upstream branch pipe 12 towards the downstream branch pipe 13 consists of a bypass flow channel which extends from the direction of advance of the liquid from the main upstream branch pipe 12 towards the chamber part 11 to one side which is substantially orthogonal to the direction of advance, after which it extends in the direction of advance of the liquid from the chamber part 11 towards the downstream branch pipe 13. In addition, an inclined surface 17, whereof the upstream portion is positioned on that one side and the downstream portion is positioned further towards the side in the direction of advance of the liquid from the chamber part 11 towards the downstream branch pipe 13 than that one side, is formed in the bypass flow channel.

## Description

### [Technical Field]

The present invention relates to a liquid coinfusion unit in which a plurality of infusion tubes or the like for medical purposes are joined and drug solutions or the like flow between all of these infusion tubes.

### [Technical Background]

Conventionally, certain drug solutions or physiological saline etc. are supplied inside the body of a patient using a plurality of infusion tubes, and in such cases all of the infusion tubes are linked in communication or blocked using a liquid coinfusion unit such as a medical stopcock (see Patent Document 1, for example). This three-way stopcock (liquid coinfusion unit) has a main body provided with three branch openings, and a first branch pipe (main upstream branch pipe), second branch pipe (downstream branch pipe) and a coinfusion opening (supplementary upstream branch pipe) which are respectively connected to each of the branch openings.

The flow channel formed from the first branch pipe as far as the second branch pipe consists of a first liquid flow channel provided on the first branch pipe side, a second liquid flow channel provided on the second branch pipe side and positioned higher up than the first flow channel, and a flow channel provided on the coinfusion opening side and connecting the downstream end of the first liquid flow channel and the upstream end of the second liquid flow channel. Consequently, liquid which is introduced from the first liquid flow channel flows to the second liquid flow channel side positioned on the upper side, and the liquid does not accumulate in the space inside the flow channels.
[Patent Document 1] Japanese Patent 3945480

### [Disclosure of the Invention]

With the conventional three-way stopcock described above, if the three-way stopcock is arranged so that the second liquid flow channel is positioned higher up than the first liquid flow channel, it is possible to prevent liquid accumulating in the space inside the flow channels. However, if the abovementioned conventional three-way stopcock is arranged so that the second liquid flow channel is positioned lower down than the first liquid flow channel, liquid which is introduced from the first liquid flow channel flows to the second liquid flow channel side which is positioned below, and in this case liquid is likely to accumulate in the space inside the flow channels.

The present invention has been devised in view of the situation outlined above, and it aims to provide a liquid coinfusion unit inside which liquid and air are unlikely to accumulate.

In order to achieve the aim described above, the structural features of the liquid coinfusion unit according to the present invention lie in the fact that it is a liquid coinfusion unit equipped with a chamber part and a main upstream branch pipe, downstream branch pipe and supplementary upstream branch pipe which are respectively connected to the chamber part; a specific liquid can flow inside a liquid flow channel which is formed from the main upstream branch pipe as far as the downstream branch pipe via the chamber part, and another liquid can flow in the liquid flow channel via a supplementary liquid flow channel which is formed from the supplementary upstream branch pipe as far as the chamber part, in which liquid coinfusion unit a portion of the liquid flow channel inside the chamber part consists of a bypass flow channel which curves from the direction of advance of the liquid from the main upstream branch pipe towards the chamber part, extending towards the supplementary upstream branch pipe, after which it extends in the direction of advance from the chamber part towards the downstream branch pipe; and an inclined surface, whereof the upstream portion is positioned on the supplementary upstream branch pipe side and the downstream portion is positioned further towards the side in the direction of advance from the chamber part towards the downstream branch pipe than the supplementary upstream branch pipe side, is formed in the bypass flow channel.

With the liquid coinfusion unit according to the present invention configured in the manner described above, the liquid flow channel running from the main upstream branch pipe to the downstream branch pipe via the chamber part does not form a straight line or a broken line which curves at one point only, rather it curves from the direction of advance inside the chamber part and extends for a time to the supplementary upstream branch pipe, after which it once again curves or bends and extends from the chamber part in the direction of advance towards the downstream branch pipe, in the direction of the line of extension of the original direction of advance, or in a direction other than the line of extension thereof. An inclined surface whereof the upstream portion is positioned on the supplementary upstream branch pipe side (a direction which curves away from the direction of advance), and the downstream portion is positioned further towards the side in the direction of advance from the chamber part towards the downstream branch pipe than the supplementary upstream branch pipe side, is formed midway along the bypass flow channel formed in this way.

For example, when the liquid flow channel running from the main upstream branch pipe to the chamber part and the liquid flow channel running from the chamber part to the downstream branch pipe are positioned on a single straight line, and the supplementary liquid flow channel running from the supplementary upstream branch pipe to the chamber part is orthogonal with respect to the liquid flow channel, the bypass flow channel separates from this straight line for a time, and forms a liquid flow channel which returns to the original straight line at the downstream side. The inclined surface is then a surface which configures part of the inner peripheral surface of the bypass flow channel. This inclined surface may be a plane or curved surface. It should be noted that the liquid flow channel running from the main upstream branch pipe to the chamber part and the liquid flow channel running from the chamber part to the downstream branch pipe need not run on a straight line, and they may each be on one of two parallel lines, or arranged at a specific angle.

According to the present invention, the upstream portion of the inclined surface is in a state in which it projects towards the inner wall of the supplementary upstream branch pipe, and this means that the space between the inner wall of the supplementary upstream branch pipe and the inclined surface becomes smaller. Consequently, if the liquid coinfusion unit is arranged so that the supplementary upstream branch pipe side is above, when liquid flowing from the main upstream branch pipe to the downstream branch pipe via the chamber part enters the chamber part from the main upstream branch pipe, it flows upwards for a time and goes down the inclined surface from the upper end of the inclined surface, and flows into the downstream branch pipe from the chamber part. As a result, the liquid flows through the upper part of the space inside the chamber part, and the air inside the chamber part does not accumulate, and no liquid accumulates inside the chamber part without flowing along the liquid flow channel. This means that there is no need for the troublesome operation to remove the air which accumulates inside the chamber part, and it is possible to suppress bacterial growth inside the chamber part.

Furthermore, if the liquid coinfusion unit is arranged so that the supplementary upstream branch pipe side is below, when liquid flowing from the main upstream branch pipe to the downstream branch pipe via the chamber part enters the chamber part from the main upstream branch pipe, it flows downwards for a time. The liquid then flows between the bottom part of the chamber part and the inclined surface, in other words the liquid goes up the inclined surface from the lower end of the inclined surface, and flows along the inclined surface to the downstream branch pipe from the chamber part. In this case too, the bypass liquid flow channel becomes a narrow flow channel because the upstream portion of the inclined surface projects towards the inner wall at the lower end of the chamber part, and there is no space where air accumulates and no space where some of the liquid accumulates inside the chamber part without flowing, and all of the liquid flows smoothly.

Furthermore, when the liquid coinfusion unit is arranged so that the supplementary upstream branch pipe side is horizontal with respect to the main upstream branch pipe and the downstream branch pipe, if the upstream side of the inclined surface is positioned above, the liquid drops down from the upstream side to the downstream side of the inclined surface, but the gap between the upstream end of the inclined surface and the inner wall of the supplementary upstream branch pipe is narrow, and therefore a space where air accumulates and a space where liquid accumulates without flowing cannot be formed between the upstream end of the inclined surface and the inner wall of the supplementary branch pipe. In addition, when the liquid coinfusion unit is arranged so that the supplementary branch pipe side is horizontal with respect to the main upstream branch pipe and the downstream branch pipe, if the upstream side of the inclined surface is positioned below, the liquid flows from the lower part of the inclined surface (upstream side) to the upper part (downstream side), and therefore a space where air accumulates and a place where liquid accumulates without flowing cannot be formed inside the chamber part, and the liquid flows smoothly.

In the same way, when the liquid coinfusion unit is arranged so that the supplementary upstream branch pipe side is horizontal with respect to the main upstream branch pipe and the downstream branch pipe, if the inclined surface is horizontally oriented, the liquid flows from the lower part towards the upper part of the chamber part while filling the space inside the chamber part, and therefore a space where air accumulates and a space where liquid accumulates without flowing cannot be formed inside the chamber part, and the liquid flows smoothly. In this way, according to the present invention, liquid and air are unlikely to accumulate inside the liquid coinfusion unit, whichever way it is oriented. In addition, according to the present invention, it is possible for a specific liquid to flow from the main upstream branch pipe to the downstream branch pipe via the chamber part, and also another liquid may flow from the supplementary upstream branch pipe to the downstream branch pipe via the chamber part.

Furthermore, another liquid may be made to mix with the specific liquid which is supplied from the main upstream branch pipe inside the chamber part, and only the other liquid may flow to the downstream branch pipe via the chamber part. Furthermore, the supplementary upstream branch pipe may consist of a pipe provided with a ceiling part comprising a rubber stopper or the like, and the inner wall of the supplementary upstream branch pipe adjacent to the upstream portion of the inclined surface may serve as the ceiling part. It should be noted that the other liquid in this case means a liquid which is not supplied from the main upstream branch pipe, and it may be the same type of liquid as the liquid supplied from the main upstream branch pipe.

Furthermore, other structural features of the liquid coinfusion unit according to the present invention lie in the fact that a portion of the bypass flow channel on the main upstream branch pipe side extends from the connecting portion between the main upstream branch pipe and the chamber part to the upstream portion of the inclined surface, and a portion of the bypass flow channel on the downstream branch pipe side extends from the downstream portion of the inclined surface to the connecting portion between the chamber part and the downstream branch pipe, and another liquid flow channel which links the supplementary upstream branch pipe and the chamber part in communication is configured by the space between the supplementary upstream branch pipe and the inclined surface. This means that it is possible to prevent the accumulation of air and some of the liquid inside the chamber part and supplementary upstream branch pipe of the liquid coinfusion unit in which two types of liquid can flow.

Further structural features of the liquid coinfusion unit according to the present invention lie in the fact that the supplementary upstream branch pipe is configured by an annular part having a circular cross section. This means that the supplementary upstream branch pipe can be simply formed.

Further structural features of the liquid coinfusion unit according to the present invention lie in the fact that the supplementary upstream branch pipe is cylindrical and is provided with an annular part having an elliptical cross section. In this case, the supplementary upstream branch pipe may consist of a single piece, or it may consist of several pieces, and part of the cross-sectional shape should be elliptical when it consists of several pieces. This means that the supplementary upstream branch pipe is easily held in the hand, which makes it simple to operate.

Further structural features of the liquid coinfusion unit according to the present invention lie in the fact that a valve body into which the injection part of a liquid injector for injecting the other liquid into the chamber part may be inserted is provided in the supplementary upstream branch pipe. This valve body may consist of a rubber stopper provided with a slit, for example. This means that the injection part of the liquid injector may consist of a male luer part, and this male luer part may be inserted through the slit in the rubber stopper so that the other liquid filling the liquid injector can be injected into the chamber part. Furthermore, a rubber stopper without a slit may be used as the valve body, and in this case the injection part of the liquid injector may be configured by a piercing needle, and this piercing needle may be made to pierce the rubber stopper to inject the other liquid filling the liquid injector into the chamber part. Once the supply of the other liquid has finished and the injection part of the liquid injector has been withdrawn from the rubber stopper, the rubber stopper returns to its original shape due to restoring force, and therefore the supplementary upstream branch pipe is closed off.

Further structural features of the liquid coinfusion unit according to the present invention lie in the fact that a stopcock is provided inside the chamber part, said stopcock forming the bypass flow channel with the chamber part, rotation of the stopcock in a specific direction making it possible to block the area between the chamber part and the main upstream branch pipe, the area between the chamber part and the downstream branch pipe, and the area between the chamber part and the supplementary upstream branch pipe, respectively. This means that it is possible to freely switch between a communicating/blocked state between the various branch pipes equipping the liquid coinfusion unit.

### [Optimum Mode of Embodiment of the Invention]

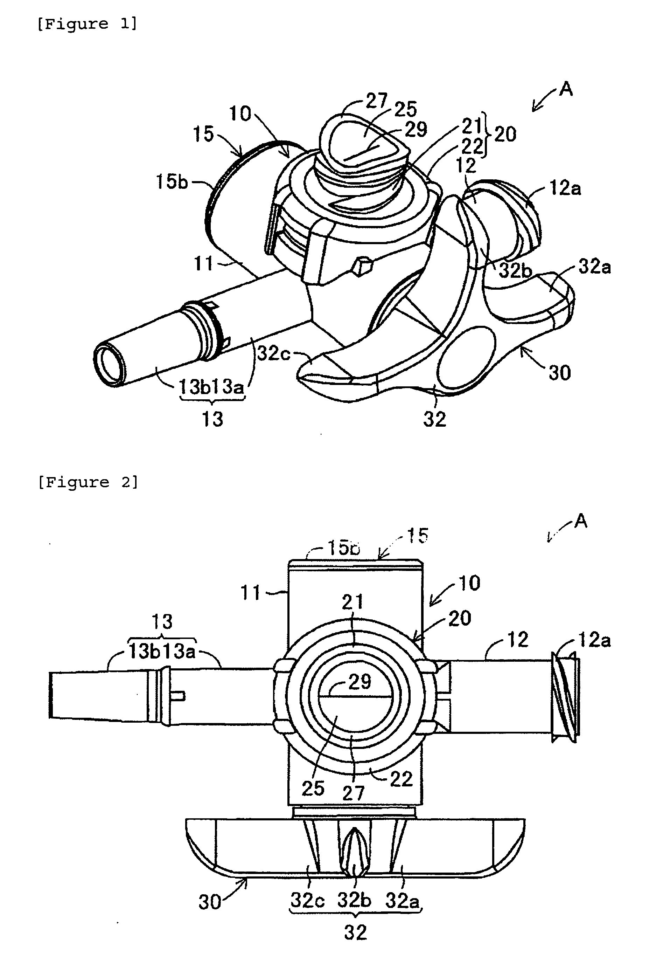

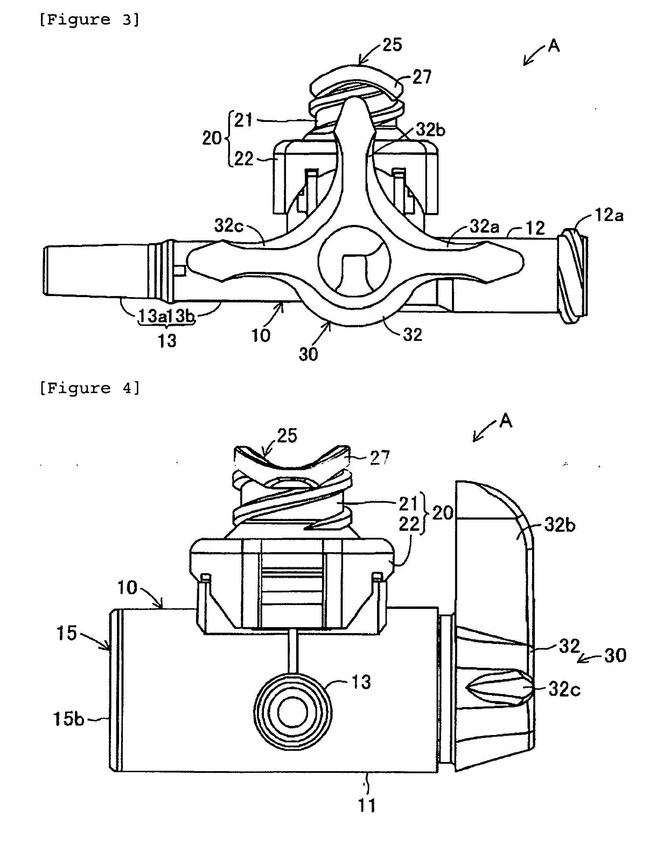

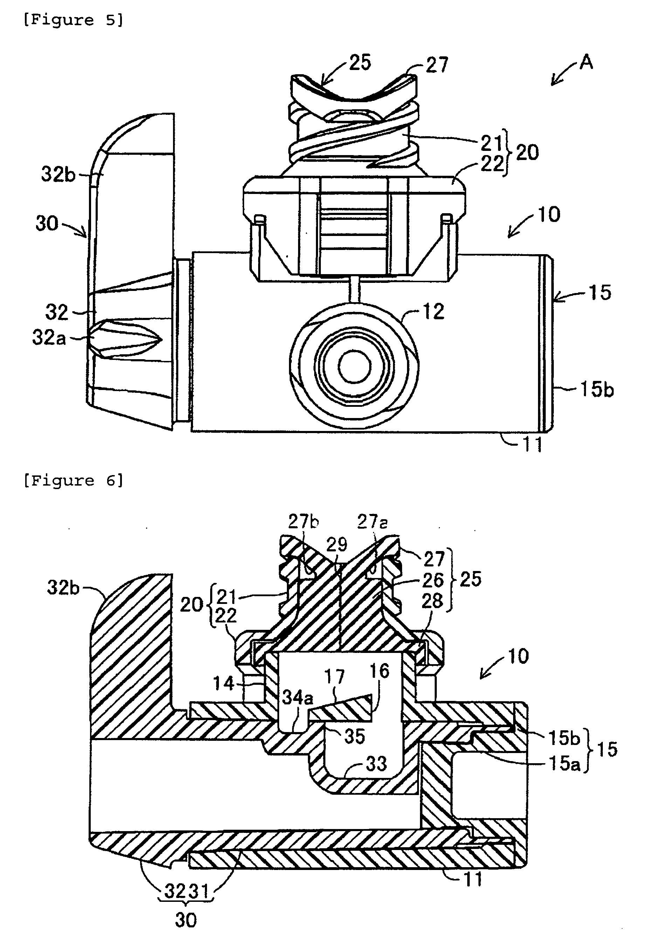

A detailed description will be given below of the liquid coinfusion unit according to one mode of embodiment of the present invention, with reference to the figures. Figures 1 to 5 show a liquid coinfusion unit A according to this mode of embodiment, and said liquid coinfusion unit A consists of a coinfusion unit main body 10, a rubber stopper 25 (the valve body according to the present invention) fitted to the coinfusion unit main body 10 with a cover member 20 interposed, and a stopcock 30. The coinfusion unit main body 10 consists of a substantially cylindrical chamber part 11 which is arranged with the axial direction going from front to rear (Figure 3 is taken as a front view; in the following description, the up-down direction and left-right direction are based on Figure 3), a main upstream branch pipe 12 and a downstream branch pipe 13 running along substantially the same axis and maintaining an angle of 180° to both the left and right sides of the chamber part 11, and a linking branch pipe 14 (see Figure 6) which is provided at the upper part of the chamber part 11, orthogonal to the main upstream branch pipe 12 and the downstream branch pipe 13.

The supplementary upstream branch pipe according to the present invention consists of the linking branch pipe 14 and the cover member 20. As shown in Figure 6, the rear opening of the chamber part 11 is closed off by a cap member 15. The cap member 15 has a shape in which the front end surface is closed off, and in which provision is made for a flange-like latch part 15b which projects outwards at the peripheral edge of the rear opening of a stepped cylindrical body 15a whereof the diameter is larger at the rear than at the front. The outer diameter of the latch part 15b is substantially equal to the outer diameter of the chamber part 11, and the outer diameter of the large-diameter portion of the stepped cylindrical body 15a is somewhat smaller than the inner diameter of the chamber part 11. The rear end of the stopcock 30 (to be described later) fits together between the outer peripheral surface of the stepped cylindrical body 15a and the inner peripheral surface of the chamber part 11.

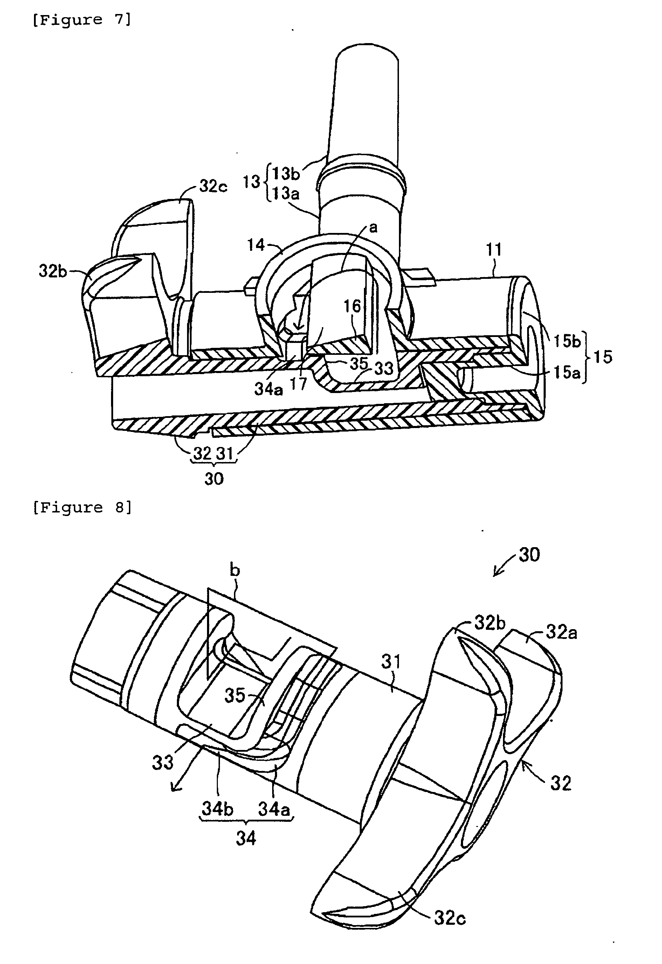

Furthermore, three communicating holes (not depicted) are formed substantially in the centre in the axial direction of the chamber part 11, spaced around the circumference 90° apart, and flow channels formed inside the main upstream branch pipe 12, linking branch pipe 14 and downstream branch pipe 13 respectively link in communication with these communicating holes. The diameter of the communicating hole which links the linking branch pipe 14 and the chamber part 11 in communication is set to be larger than the diameters of the other communicating holes. Furthermore, as shown in Figures 6 and 7, a dividing wall 16 for dividing into front and rear the flow channel running from the main upstream branch pipe 12 towards the downstream branch pipe 13 is formed in a bridging state on both sides in the left-to-right direction (the portion from the front of the page to the rear in Figure 6) on the inner wall of the communicating hole which links the chamber part 11 and the linking branch pipe 14 in communication.

As shown by the arrow a in Figure 7, the flow channel running from the main upstream branch pipe 12 towards the downstream branch pipe 13 extends upwards at the rear of the chamber part 11 from the main upstream branch pipe 12, after which it extends to the front of the chamber part 11 along the upper surface of the dividing wall 16, and then extends from the lower region at the front of the chamber part 11 to the downstream branch pipe 13. The upper surface of the dividing wall 16 is formed with an inclined surface 17 whereof the rear part is high and the front part is low, and the space above the high portion of the inclined surface 17 links in communication with the flow channel of the main upstream branch pipe 12, and the space above the low portion of the inclined surface 17 links in communication with the flow channel of the downstream branch pipe 13. Furthermore, the main upstream branch pipe 12 is formed as a single piece with the chamber part 11, and has a flow channel formed inside which comprises a tapered hole part in which the chamber part 11 side is narrower in diameter than the upstream opening. A linking thread part 12a is then formed on the outer peripheral surface of the opening of the main upstream branch pipe 12.

The downstream branch pipe 13 is formed as a single piece with the chamber part 11, and it consists of a base end part 13a on the chamber part 11 side, and a male luer part 13b on the tip end side which is formed to be narrower than the base end part 13a. Furthermore, the male luer part 13b is formed with a tapering shape in which the tip end portion is narrower than the portion at the base end part 13a side. The linking branch pipe 14 consists of a cylindrical part which is short in the axial direction and which is provided at the upper part of the chamber part 11, and a cover member 20 for fixing the rubber stopper 25 to the linking branch pipe 14 is fitted to the outer peripheral surface of this linking branch pipe 14.

The cover member 20 has a substantially cylindrical shape with steps of varying diameter, and it has a substantially cap-like shape in which the upper part 21 is somewhat smaller in diameter than the linking branch pipe 14, and the lower part 22 is larger in diameter than the linking branch pipe 14. The lower end on the inner peripheral surface of the lower part 22 of the cover member 20 is then made to engage with the outer peripheral surface of the linking branch pipe 14 so that it is detachably fitted to the linking branch pipe 14. Furthermore, a prescribed gap is formed at the upper part of the engagement part between the lower part 22 of the cover member 20 and the linking branch pipe 14. This gap serves to fix the lower end part of the rubber stopper 25 to the cover member 20.

The rubber stopper 25 is made of an elastic member which can expand and contract, such as natural rubber, synthetic rubber or an elastomer, and it consists of a thick disc-shaped rubber stopper main body 26, a flange-like engaging part 27 which is formed at the upper part of the rubber stopper main body 26, and a substantially cylindrical fixing piece 28 which is formed at the lower part of the rubber stopper main body 26. The flange-like engaging part 27 is formed with notches 27a, 27b between the two side portions at the upper part of the rubber stopper main body 26, and it is substantially disc-shaped, joining to the rubber stopper main body 26 at the central portion, and its diameter is somewhat larger than the diameter of the rubber stopper main body 26. The fixing piece 28 consists of a projecting piece which projects from the lower part of the rubber stopper main body 26.

As shown in Figure 6, the rubber stopper main body 26 and the fixing piece 28 of the rubber stopper 25 are pushed inside the cover member 20, and the flange-like engaging part 27 is made to engage with the opening edge part of the cover member 20, and also the fixing piece 28 is gripped between the lower part 22 of the cover member 20 and the linking branch pipe 14, whereby the rubber stopper 25 is fixed to the cover member 20. In this way, the flange-like engaging part 27 engages with the open edge of the cover member 20 so that the rubber stopper 25 is prevented from entering the inside of the cover member 20, and the fixing piece 28 is fixed by the lower part 22 of the cover member 20 and the linking branch pipe 14, thereby preventing the rubber stopper 25 from being withdrawn upwards from the cover member 20.

By means of this, the rubber stopper main body 26 is firmly pressured, whereby the flange-like engaging part 27 extends, allowing said rubber stopper main body to move from the upper part 21 into the lower part 22 of the cover member 20. Furthermore, when the rubber stopper main body 26 moves downwards, the fixing piece 28 and the region of the linking part between the rubber stopper main body 26 and fixing piece 28 also extend. Furthermore, a slit 29 which passes through the area between the inside of the linking branch pipe 14 and the outside of the cover member 20, and linking the flow channel inside the linking branch pipe 14 in communication with the outside, is provided in the rubber stopper main body 26 of said rubber stopper 25.

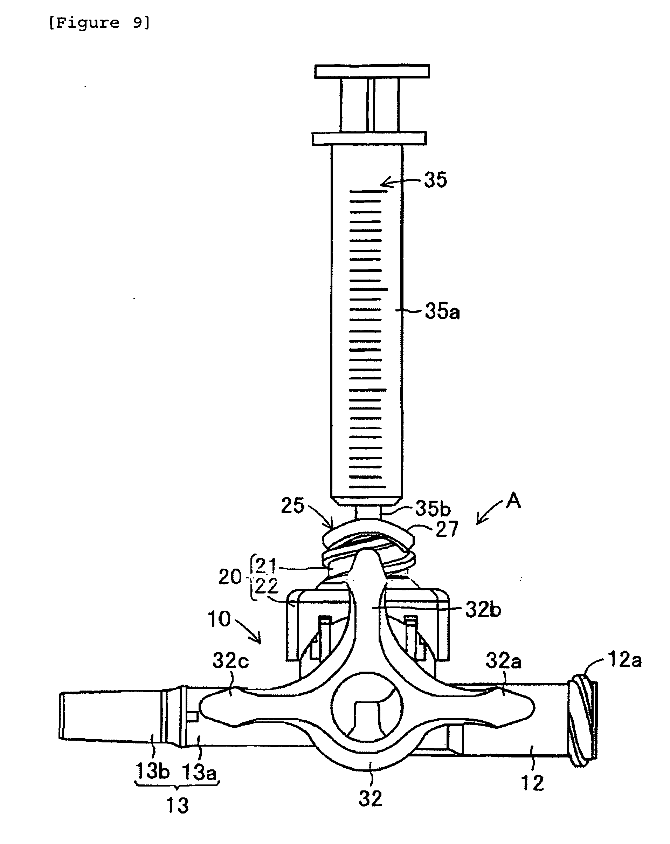

This slit 29 remains closed off due to the elasticity of the rubber stopper main body 26 when the flow channel inside the linking branch pipe 14 (supplementary upstream branch pipe) is not being used. Furthermore, when the flow channel inside the linking branch pipe 14 is being used, a male luer part 35b (see Figure 9) of a syringe 35 serving as the liquid injector according to the present invention, for example, is inserted into the slit 29 of the rubber stopper 25, whereby a drug solution housing part 35a of the syringe 35 and the flow channel of the linking branch pipe 14 can be made to communicate. At this time, the area between the male luer part 35b and the slit 29 are in tight contact because of the elasticity of the rubber stopper main body 26. The flow channel of the supplementary upstream branch pipe is formed by the space inside the linking branch pipe 14 and the slit 29 of the rubber stopper 25.

As shown in Figure 8, the stopcock 30 consists of a substantially cylindrical stopcock main body 31 and an operating part 32 which is linked to the front end of the stopcock main body 31. The stopcock main body 31 is then disposed inside the chamber part 11 in a state in which the tip end (rear end) is inserted between the inner peripheral surface of the chamber part 11 and the outer peripheral surface of the stepped cylindrical body 15a of the cap member 15, and rotation of the operating part 32 causes rotation in the axial direction of the chamber part 11. Furthermore, two groove parts 33, 34 are arranged in the axial direction on the outer peripheral surface of the stopcock main body 31. The groove part 33 consists of a recess running along the circumference, substantially over a semicircle, at a portion somewhat further to the rear than the centre in the axial direction on the outer peripheral surface of the stopcock main body 31.

Furthermore, the groove part 34 consists of a substantially L-shaped recess comprising: a circumferential-direction groove part 34a which runs parallel to the groove part 33 along the outer peripheral surface of the stopcock main body 31, at a portion somewhat further forwards than the centre in the axial direction on the outer peripheral surface of the stopcock main body 31; and an axial-direction groove part 34b which curves from one end of the circumferential-direction groove part 34a and runs towards the rear side. The axial-direction groove part 34b of the groove part 34 is provided at a position which maintains a prescribed spacing with one end of the groove part 33, and the other end of the circumferential-direction groove part 34a of the groove part 34 is positioned more towards one side in the circumferential direction than the other end of the groove part 33 (the front in Figure 8).

The length of the groove part 33 and the groove part 34 along the circumferential direction of the stopcock main body 31 is set to be equal to substantially a semicircle in both cases, and the gap between the groove part 33 and the circumferential-direction groove part 34a of the groove part 34, and the gap between the groove part 33 and the axial-direction groove part 34b of the groove part 34 are set to be substantially equal. A barrier part 35 which runs along the outer peripheral surface of the stopcock main body 31 is formed between the groove part 33 and the circumferential-direction groove part 34a of the groove part 34. Furthermore, the portion of the stopcock main body 31 disposed inside the chamber part 11 where the groove part 33 and the axial-direction groove part 34b of the groove part 34 are formed on the outer peripheral surface matches the position of the communication hole which links the chamber part 11 and main upstream branch pipe 12 in communication, and the communicating hole which links the chamber part 11 and downstream branch pipe 13 in communication; and the circumferential-direction groove part 34a of the groove part 34 is opposite the front portion on the inner peripheral surface of the chamber part 11.

Furthermore, the portion on the outer peripheral surface of the stopcock main body 31 where the barrier part 35 is formed is opposite the front end portion of the inner surface of the dividing wall 16 on the inner peripheral surface of the chamber part 11. Consequently, as shown in Figure 7, when the stopcock main body 31 is positioned so that the barrier part 35 is upwards, the groove part 33 is opposite the flow channel inside the main upstream branch pipe 12 (positioned in front in Figure 7), and the inside of the chamber part 11 and the flow channel of the main upstream branch pipe 12 communicate via the groove part 33. Furthermore, the rear part of the axial-direction groove part 34b of the groove part 34 (positioned to the rear of the groove part 33 in Figure 7) is opposite the flow channel inside the downstream branch pipe 13, and the inside of the chamber part 11 and the flow channel of the downstream branch pipe 13 communicate via the groove part 34.

In this case, the dividing wall 16 is positioned above the barrier part 35, and the upper surface of the barrier part 35 and the lower surface of the dividing wall 16 are substantially in tight contact. The space forming the flow channel of the linking branch pipe 14 lies above the dividing wall 16, and therefore the groove part 33 and the groove part 34 communicate via the flow channel of the linking branch pipe 14. Accordingly, in this state, a liquid such as a drug solution can flow from the main upstream branch pipe 12 to the downstream branch pipe 13 via the chamber part 11 and the linking branch pipe 14. In this case, as shown by the arrow b in Figure 8, a drug solution or the like which flows from the main upstream branch pipe 12 into the groove part 33 goes over the top of the dividing wall 16 (positioned at the upper portion of the arrow b) and flows to the groove part 34.

At this time, the inclined surface 17 which is formed on the upper surface of the dividing wall 16 is high at the rear and low at the front, and therefore the drug solution or the like passes through bypassing the space inside at the top of the chamber part 11, and it is possible to suppress the phenomenon of air and some of the drug solution etc. accumulating inside the chamber part 11 and the linking branch pipe 14. When the stopcock 30 is turned in one direction from this state, causing the groove part 33 to lie opposite the flow channel of the downstream branch pipe 13, and the outer peripheral surface of the stopcock main body 31 is made to lie opposite the flow channel of the main upstream branch pipe 12, the area between the inside of the chamber part 11 and the downstream branch pipe 13 is linked in communication, and the area between the inside of the chamber part 11 and the main upstream branch pipe 12 is blocked.

Furthermore, when the stopcock 30 is turned in the other direction from the state shown in Figure 6, causing one end portion of the groove part 33 to lie opposite the flow channel of the main upstream branch pipe 12 and maintaining the state of communication, and the outer peripheral surface of the stopcock main body 31 is made to lie opposite the flow channel of the downstream branch pipe 13, the area between the inside of the chamber part 11 and the downstream branch pipe 13 is blocked, and the area between the inside of the chamber part 11 and the main upstream branch pipe 12 is linked in communication. By turning the stopcock 30 in this way, it is possible to link both the main upstream branch pipe 12 and the downstream branch pipe 13 in communication with the inside of the chamber part 11, and to block one of them only from the inside of the chamber part 11.

Moreover, the operating part 32 comprises three operating pieces 32a, 32b, 32c which are formed at an angle of 90° to one another, so that said operating pieces 32a, 32b, 32c correspond to the main upstream branch pipe 12, linking branch pipe 14 and downstream branch pipe 13, respectively. That is to say, as shown in Figure 3, when the operating piece 32a is in a position pointing to the main upstream branch pipe 12, when the operating piece 32b is in a position pointing to the linking branch pipe 14 (supplementary upstream branch pipe), and when the operating piece 32c is in a position pointing to the downstream branch pipe 13, the main upstream branch pipe 12, linking branch pipe 14 and downstream branch pipe 13 are all in a state of communication via the chamber part 11.

Furthermore, when the operating part 32 is turned through 90° in a clockwise direction from the state shown in Figure 3 so that the operating piece 32b is in a position pointing to the main upstream branch pipe 12 and the operating piece 32c is in a position pointing to the linking branch pipe 14, the main upstream branch pipe 12 and the linking branch pipe 14 are in communication, and the area between the linking branch pipe 14 and the downstream branch pipe 13 is in a blocked state. In addition, when the operating part 32 is turned through 90° in an anti-clockwise direction from the state shown in Figure 3 so that the operating piece 32a is in a position pointing to the linking branch pipe 14 and the operating piece 32b is pointing to the downstream branch pipe 13, the linking branch pipe 14 and the downstream branch pipe 13 are in communication, and the area between the main upstream branch pipe 12 and the linking branch pipe 14 is in a blocked state.

Furthermore, as shown in Figure 9, a syringe 35 can be detachably fitted into the cover member 20 of the supplementary upstream branch pipe, via the rubber stopper 25. This syringe 35 comprises a drug solution housing part 35a for housing a drug solution or the like, and a cylindrical male luer part 35b of narrow diameter, and the male luer part 35b is inserted into the slit 29 of the rubber stopper 25, thereby allowing the inside of the drug solution housing part 35a and the flow channel of the linking branch pipe 14 to be linked in communication. When this male luer part 35b is inserted into the slit 29 of the rubber stopper 25, the male luer part 35b makes the slit 29 expand, while the rubber stopper main body 26 is moved so that it presses against the inner peripheral surface of the linking branch pipe 14. At this time, the linking part between the rubber stopper main body 26 and the fixing piece 28 extends downwards.

By means of this, the rubber stopper main body 26 is pressed against the inner peripheral surface of the linking branch pipe 14 by the pressure from the male luer part 35b, and said rubber stopper main body moves down in a state in which it is in tight contact with the inner peripheral surface of the linking branch pipe 14. Then, when the male luer part 35b is inserted up to a suitable state inside the slit 29, the lower surface of the rubber stopper main body 26 reaches a state in which it is in close proximity to the inclined surface 17 of the dividing wall 16. Furthermore, the area between the inner peripheral surface of the linking branch pipe 14 and the rubber stopper main body 26, and the area between the male luer part 35b and the peripheral surface of the slit 29 are in a state of tight contact because of the elasticity of the rubber stopper 25. Consequently, the main upstream branch pipe 12 and the downstream branch pipe 13 are linked in communication so that while a drug solution or the like flows from the main upstream branch pipe 12 towards the downstream branch pipe 13, another drug solution or the like may be mixed with said drug solution or the like from the syringe 35. Furthermore, when the area between the chamber part 11 and the main upstream branch pipe 12 is in a blocked state, another drug solution or the like may also flow from the syringe 35 to the downstream branch pipe 13.

When two types of drug solution are supplied to a patient's body (not depicted) with this configuration, the rear end of a transfusion tube (not depicted) to which an indwelling needle for piercing the patient and remaining indwelling is first of all connected to the downstream branch pipe 13. Next, the male luer part provided at the tip end of the transfusion tube extending from a container or the like for housing the first drug solution which is to be supplied to the patient is connected to the main upstream branch pipe 12. The male luer part 35b is then passed through the slit 29 in the rubber stopper 25 in a state in which the other drug solution is sucked into the drug housing part 35a of the syringe 35.

The first drug solution then passes into the transfusion line which includes the chamber part 11, and all of the air in the transfusion line is expelled to the outside, after which the drug solution in the container or the like is delivered to the patient in a state in which the indwelling needle has pierced the patient and lies indwelling, whereby the drug solution is supplied to the patient. Furthermore, the other drug solution inside the drug solution housing part 35a of the syringe 35 is also appropriately delivered into the chamber part 11 via the flow channel of the linking branch pipe 14. In this case, the area between the inner peripheral surface of the linking branch pipe 14 and the rubber stopper 26, and the area between the male luer part 35b and the peripheral surface of the slit 29 are respectively in a state of tight contact. Furthermore, the lower surface of the rubber stopper main body 26 is in close proximity to the inclined surface 17 of the dividing wall 16, and therefore there is only a small amount of space inside the chamber part 11 and the linking branch pipe 14, and there is unlikely to be a place where air and some of the drug solution can accumulate inside the chamber part 11 and the linking branch pipe 14.

That is to say, all of the space inside the chamber part 11 and the linking branch pipe 14 forms a flow channel for the drug solution, and therefore air is unlikely to accumulate. This means that it is possible to suppress the phenomenon of air being mixed in with the drug solution which is supplied to the patient. Furthermore, it is no longer the case that some of the drug solution accumulates inside the chamber part 11 and the linking branch pipe 14. Then, when the supply of drug solution from the syringe 35 is finished, and the male luer part 35b has been withdrawn from the slit 29, the rubber stopper main body 26 is released from the pressure applied by the male luer part 35b, which takes us back to the state shown in Figure 6, due to the restoring force of the flange-like engaging part 27 and the fixing piece 28. Furthermore, by means of this liquid coinfusion unit A, the inside of the cover member 20 fitted to the linking branch pipe 14 is closed off by the rubber stopper 25, and therefore it is possible to suppress the entry of air into the chamber part 11 from the supplementary upstream branch pipe, and bacterial growth.

In this way, with the liquid coinfusion unit A according to this mode of embodiment, the flow channel running from the main upstream branch pipe 12 to the downstream branch pipe 13 via the chamber part 11, is oriented upwards for a time inside the chamber part 11, extending to the upper surface of the dividing wall 16, after which it goes down the inclined surface 17 formed on the upper surface of the dividing wall 16, and links in communication with the flow channel of the downstream branch pipe 13. Consequently, when drug solution flowing from the main upstream branch pipe 12 to the downstream branch pipe 13 via the chamber part 11 enters the chamber part 11 from the main upstream branch pipe 12, it flows upwards for a time, and then goes down the inclined surface 17 from the upper end of the inclined surface 17, and flows from the lower portion of the chamber part 11 to the downstream branch pipe 13.

As a result, the drug solution flows through the upper part of the space inside the chamber part 11 and the linking branch pipe 14, and air inside the chamber part 11 and the linking branch pipe 14 no longer accumulates, and also drug solution inside the chamber part 11 and the linking branch pipe 14 no longer accumulates. As a result, there is no need for the troublesome operation to remove air which has accumulated inside the chamber part 11 and the linking branch pipe 14, and it is also possible to suppress bacterial growth inside the chamber part 11 and the linking branch pipe 14.

Furthermore, if the liquid coinfusion unit A is vertically inverted so that the linking branch pipe 14 is below the chamber part 11, when drug solution flowing from the main upstream branch pipe 12 to the downstream branch pipe 13 via the chamber part 11 enters the chamber part 11 from the main upstream branch pipe 12, it flows downwards for a time. The drug solution then flows upwards between the lower end of the chamber part 11 and the inclined surface 17, going up the inclined surface 17 from the lower end of the inclined surface 17, and flows from the upper portion of the chamber part 11 to the downstream branch pipe 13.

In this case too, the upstream portion of the inclined surface 17 projects towards the inner wall of the chamber part 11, and therefore there is no space where air accumulates inside the chamber part 11, and no space where the drug solution accumulates without flowing, and the drug solution flows smoothly. Furthermore, in the same way, when the liquid coinfusion unit A is arranged on its side so that the linking branch pipe 14 is horizontal with respect to the main upstream branch pipe 12 and the downstream branch pipe 13, a space where air accumulates and a space where drug solution accumulates without flowing cannot be formed inside the chamber part 11, and the drug solution flows smoothly. In this way, it is possible to prevent the accumulation of air and drug solution therein, however the liquid coinfusion unit A is arranged.

Furthermore, the liquid coinfusion unit A is provided with a supplementary upstream branch pipe comprising the linking branch pipe 14 etc., and therefore not only is it possible for a specific drug solution to flow from the main upstream branch pipe 12 to the downstream branch pipe 13 via the chamber part 11, it is also possible for another drug solution to flow from the linking branch pipe 14 to the downstream branch pipe 13 via the chamber part 11. Furthermore, with the liquid coinfusion unit A, provision is made for a rubber stopper 25 furnished with a slit 29 inside the cover member 20 which is fitted to the linking branch pipe 14, and therefore the male luer part 35b of the syringe 35 can be inserted into the slit 29 of the rubber stopper 25 so that another drug solution which fills the syringe 35 can be injected into the chamber part 11. In addition, the liquid coinfusion unit A is furnished with a stopcock 30, and therefore the area between the chamber part 11 and the main upstream branch pipe 12, and the area between the chamber part 11 and the downstream branch pipe 13 can be freely linked in communication or blocked, respectively.

### (Variant Example 1)

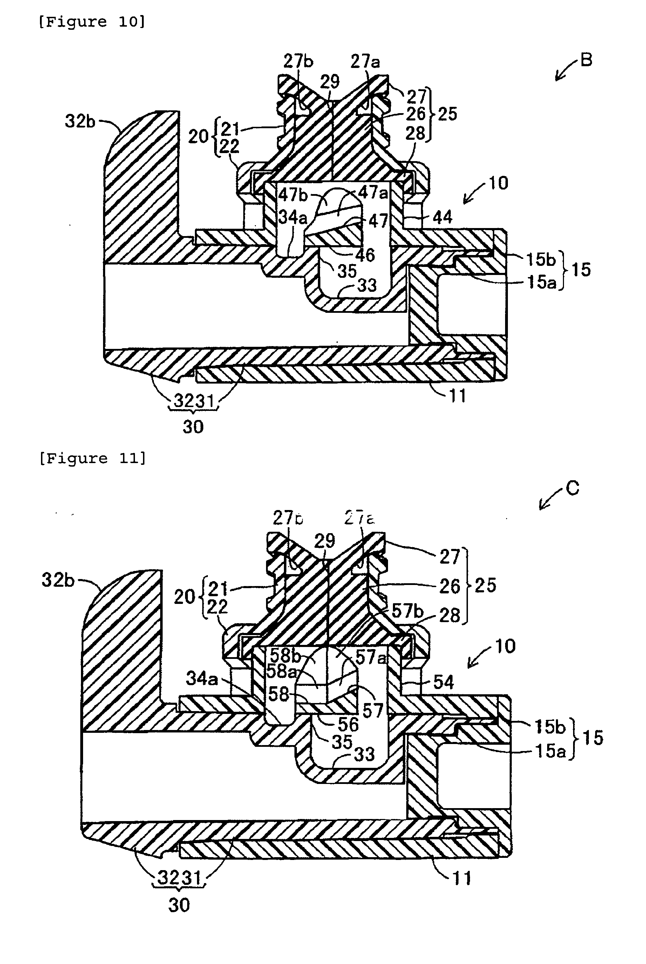

Figure 10 shows a liquid coinfusion unit B according to Variant Example 1 of the liquid coinfusion unit A described above. With this liquid coinfusion unit B, inclined parts are also formed at both the left- and right-hand portions, rather than having a plane surface in which an inclined surface 47 formed at the upper surface of a dividing wall 46 is simply high at the rear and low at the front. That is to say, auxiliary inclined surfaces 47a, 47b having a two-step form which becomes steadily higher towards the left are formed on the left-hand portion of the inclined surface 47 (portion on the downstream branch pipe side), and auxiliary inclined surfaces (not depicted) having a two-step form which becomes steadily higher towards the right are formed on the right-hand portion of the inclined surface 47 (portion on the main upstream branch pipe side).

The auxiliary inclined surfaces 47a, 47b and the two-step auxiliary inclined surfaces formed on the right-hand portion of the inclined surface 47 are formed to be laterally symmetrical, and the auxiliary inclined surface 47a etc. positioned below is inclined upwards towards the inner peripheral surface of a linking branch pipe 44 from the edge of the plane portion in the centre of the inclined surface 47. The auxiliary inclined surface 47b etc. positioned above extends towards the inner surface of the linking branch pipe 44 from the upper edge of the auxiliary inclined surface 47a etc., and the angle of inclination of the auxiliary inclined surface 47b etc. is greater than the angle of inclination of the auxiliary inclined surface 47a etc. The other structural components of this liquid coinfusion unit B are the same as those of the liquid coinfusion unit A described above. Accordingly, like components bear the same reference symbols, and they will not be described again.

With this liquid coinfusion unit B, the space inside the linking branch pipe 44 is small, and therefore it is possible to more reliably eliminate a space where air accumulates and a space where drug solution accumulates without flowing inside the chamber part 11 and the linking branch pipe 44. Furthermore, the auxiliary inclined surfaces 47a, 47b etc. are provided on both the left- and right-hand sides of the inclined surface 47, and therefore the drug solution or the like flowing over the inclined surface 47 collects in the centre of the inclined surface 47, and the drug solution or the like flows smoothly. Furthermore, by providing the auxiliary inclined surfaces 47a, 47b, it is possible to prevent a sharp angle (a right angle or an angle close to that) from being formed between the inclined surface 47 and the inner peripheral surface of the linking branch pipe 44. This means that air bubbles and drug solution are unlikely to accumulate between the inclined surface 47 and the inner peripheral surface of the linking branch pipe 44. The other operational effects of this liquid coinfusion unit B are the same as the operational effects of the liquid coinfusion unit A described above.

### (Variant Example 2)

Figure 11 shows a liquid coinfusion unit C according to Variant Example 2 of the liquid coinfusion unit A described above. With this liquid coinfusion unit C, the upper surface of a dividing wall 56 consists of an inclined surface 57 formed to the rear and a horizontal surface 58 formed to the front. Respective inclined parts are then formed at both the left- and right-hand portions of the inclined surface 57 and the horizontal surface 58. That is to say, auxiliary inclined surfaces 57a, 57b having a two-step form which becomes steadily higher towards the left are formed on the left-hand portion of the inclined surface 57 (portion on the downstream branch pipe side), and auxiliary inclined surfaces (not depicted) having a two-step form which becomes steadily higher towards the right are formed on the right-hand portion of the inclined surface 57 (portion on the main upstream branch pipe side).

Furthermore, auxiliary inclined surfaces 58a, 58b having a two-step form which becomes steadily higher towards the left are formed on the left-hand portion of the horizontal surface 58 (portion on the downstream branch pipe side), and auxiliary inclined surfaces (not depicted) having a two-step form which becomes steadily higher towards the right are formed on the right-hand portion of the horizontal surface 58 (portion on the main upstream branch pipe side). The auxiliary inclined surfaces 57a, 57b are inclined in the front-to-rear direction and left-to-right direction, while the auxiliary inclined surfaces 58a, 58b are inclined in the left-to-right direction only. Furthermore, the auxiliary inclined surfaces 57a, 57b and the auxiliary inclined surfaces having a two-step form which are formed on the right-hand portion of the inclined surface 57 are formed to be laterally symmetrical, and the auxiliary inclined surfaces 58a, 58b and the auxiliary inclined surfaces having a two-step form which are formed on the right-hand portion of the horizontal surface 58 are formed to be laterally symmetrical.

The auxiliary inclined surface 57a etc. is inclined upwards towards a linking branch pipe 54 from the edge of the central portion of the inclined surface 57. The auxiliary inclined surface 57b extends towards the inner surface of the linking branch pipe 54 from the upper edge of the auxiliary inclined surface 57a etc., and the angle of inclination of the auxiliary inclined surface 57b etc. is greater than the angle of inclination of the auxiliary inclined surface 57a etc. In the same way, the auxiliary inclined surface 58a etc. is inclined upwards towards the linking branch pipe 54 from the edge of the central portion of the horizontal surface 58. The auxiliary inclined surface 58b etc. extends towards the inner surface of the linking branch pipe 54 from the upper edge of the auxiliary inclined surface 58a etc., and the angle of inclination of the auxiliary inclined surface 58b etc. is greater than the angle of inclination of the auxiliary inclined surface 58a etc. The other structural components of this liquid coinfusion unit C are the same as those of the liquid coinfusion unit A described above. Accordingly, like components bear the same reference symbols, and they will not be described again.

With this liquid coinfusion unit C, the auxiliary inclined surfaces 57a, 57b etc. are provided on both the left- and right-hand sides of the inclined surface 57, and therefore the same effect can be achieved as with the liquid coinfusion unit B described above, namely an effect whereby a drug solution or the like flowing over the inclined surface 57 is made to collect in the centre of the inclined surface 57 and the drug solution or the like flows smoothly. Furthermore, by providing the auxiliary inclined surfaces 57a, 57b, and the auxiliary inclined surfaces 58a, 58b, it is possible to prevent a sharp angle (a right angle or an angle close to that) from being formed between the inclined surface 57 and horizontal surface 58, and the inner peripheral surface of the linking branch pipe 54. It is also possible to use a liquid coinfusion unit in which the branch pipes consist only of the main upstream branch pipe 12 and the downstream branch pipe 13, without providing a supplementary upstream branch pipe.

This means that air bubbles and drug solution are unlikely to accumulate between the inclined surface 57 and horizontal surface 58, and the inner peripheral surface of the linking branch pipe 54. In addition, by providing the horizontal surface 58 on the upper surface of the dividing wall 56, it is possible to provide the inclined surface 57 while maintaining a space for the rubber stopper 25 to open when the male luer part 35b of the syringe 35 is inserted into the slit 29 of the rubber stopper 25. The other operational effects of this liquid coinfusion unit C are the same as the operational effects of the liquid coinfusion unit A described above.

Furthermore, the liquid coinfusion unit according to the present invention is not limited to the modes of embodiment described above, and appropriate modifications may be made. For example, in the modes of embodiment described above, a system provided with the stopcock 30 is used as the liquid coinfusion unit, but it is also possible to use, as the liquid coinfusion unit according to the present invention, a system without the stopcock 30, in which a drug solution or the like is made to flow from the main upstream branch pipe 12 to the downstream branch pipe 13 which is normally in communication therewith, and also in which another drug solution or the like can flow from the supplementary upstream branch pipe to the chamber part 11.

Furthermore, in the modes of embodiment described above, the cover member 20 is formed with a substantially cap-like shape which is circular when seen from above, but this cover member may be formed with a cap-like shape which is elliptical or oval when seen from above. This makes the liquid coinfusion unit easy to hold with the hand. In this case, the linking branch pipe may be made cylindrical, and the cover member may be made to have a cap-like shape which is elliptical or oval when seen from above. In addition, it is also possible to use a rubber stopper 25 without the slit 29, and to supply another drug solution or the like into the chamber part using a syringe provided with a piercing needle. Furthermore, the shape of the inclined surface formed on the upper surface of the dividing wall may also be suitably modified.

### [Brief Description of the Figures]

[Figure 1] is an oblique view showing the liquid coinfusion unit according to a mode of embodiment of the present invention;
[Figure 2] is a planar view of the liquid coinfusion unit;
[Figure 3] is a front view of the liquid coinfusion unit;
[Figure 4] is a view of the liquid coinfusion unit from the left-hand side;
[Figure 5] is a view of the liquid coinfusion unit from the right-hand side;
[Figure 6] is a view of the liquid coinfusion unit in cross section;
[Figure 7] is a cutaway view in cross section showing the inside of the liquid coinfusion unit;
[Figure 8] is an oblique view showing the stopcock;
[Figure 9] is a front view showing a state in which the syringe is fitted in the liquid coinfusion unit;
[Figure 10] is a view in cross section showing the liquid coinfusion unit according to Variant Example 1; and
[Figure 11] is a view in cross section showing the liquid coinfusion unit according to Variant Example 2.

### [Key to Symbols]

11...chamber part; 12...main upstream branch pipe; 13...downstream branch pipe; 14, 44, 54...linking branch pipe; 16, 46, 56...dividing wall; 17, 47, 57...inclined surface; 20...cover member; 25...rubber stopper; 30...stopcock; 35...syringe; 35b...male luer part; 47a, 47b, 57a, 57b, 58a, 58b...auxiliary inclined surface; 58...horizontal surface; A, B, C...liquid coinfusion unit.

## Claims

**1.** Liquid coinfusion unit equipped with a chamber part and a main upstream branch pipe, downstream branch pipe and supplementary upstream branch pipe which are respectively connected to the abovementioned chamber part; a specific liquid can flow inside a liquid flow channel which is formed from the abovementioned main upstream branch pipe as far as the abovementioned downstream branch pipe via the abovementioned chamber part, and another liquid can flow in the abovementioned liquid flow channel via a supplementary liquid flow channel which is formed from the abovementioned supplementary upstream branch pipe as far as the abovementioned chamber part,
said liquid coinfusion unit being **characterized in that** a portion of the abovementioned liquid flow channel inside the abovementioned chamber part consists of a bypass flow channel which curves from the direction of advance of the abovementioned liquid from the abovementioned main upstream branch pipe towards the abovementioned chamber part, extending towards the abovementioned supplementary upstream branch pipe, after which it extends in the direction of advance from the abovementioned chamber part towards the abovementioned downstream branch pipe; and
an inclined surface, whereof the upstream portion is positioned on the abovementioned supplementary upstream branch pipe side and the downstream portion is positioned further towards the side in the direction of advance from the abovementioned chamber part towards the abovementioned downstream branch pipe than the abovementioned supplementary upstream branch pipe side, is formed in the abovementioned bypass flow channel.

**2.** Liquid coinfusion unit according to Claim 1, in which a portion of the abovementioned bypass flow channel on the abovementioned main upstream branch pipe side extends from the connecting portion between the abovementioned main upstream branch pipe and the abovementioned chamber part to the upstream portion of the abovementioned inclined surface, and a portion of the abovementioned bypass flow channel on the abovementioned downstream branch pipe side extends from the downstream portion of the abovementioned inclined surface to the connecting portion between the abovementioned chamber part and the abovementioned downstream branch pipe, and another liquid flow channel which links the abovementioned supplementary upstream branch pipe and the abovementioned chamber part in communication is configured by the space between the abovementioned supplementary upstream branch pipe and the abovementioned inclined surface.

**3.** Liquid coinfusion unit according to Claim 1 or 2, in which the abovementioned supplementary upstream branch pipe is configured by an annular part having a circular cross section.

**4.** Liquid coinfusion unit according to Claim 1 or 2, in which the abovementioned supplementary upstream branch pipe is cylindrical and is provided with an annular part having an elliptical cross section.

**5.** Liquid coinfusion unit according to any one of Claims 1 to 4, in which a valve body into which the injection part of a liquid injector for injecting the abovementioned other liquid into the abovementioned chamber part may be inserted is provided in the abovementioned supplementary upstream branch pipe.

**6.** Liquid coinfusion unit according to any one of Claims 1 to 5, in which a stopcock is provided inside the abovementioned chamber part, said stopcock forming the abovementioned bypass flow channel with the abovementioned chamber part, rotation of the stopcock in a specific direction making it possible to block the area between the abovementioned chamber part and the abovementioned main upstream branch pipe, the area between the abovementioned chamber part and the abovementioned downstream branch pipe, and the area between the abovementioned chamber part and the abovementioned supplementary upstream branch pipe, respectively.
